# EUROPÄISCHE PATENTANMELDUNG

(11) **EP 0 775 475 A2**
(43) Veröffentlichungstag der Anmeldung: **28.05.1997**
(21) Anmeldenummer: 96116093.4
(22) Anmeldetag: 08.10.1996
(51) Int. Cl.: A61F 2/34

(54) **Schalenförmige Pfanne für eine Hüftgelenkprothese**

(30) Priorität: 22.11.1995 DE 19543545
(71) Anmelder: ENDOCARE AG, CH-6343 Rotkreuz (CH)
(72) Erfinder: Lintner, F. Prof. Dr., 1180 Wien (AT)
(74) Vertreter: Popp, Eugen, Dr.

(57) **Zusammenfassung**

Es wird eine schalenförmige, insbesondere halbkugelförmige Pfanne für eine Hüftgelenkprothese zum Einsatz im Acetabulum mit einer Vielzahl von Verankerungselementen (11) an ihrer Außenfläche (12) beschrieben, wobei zumindest die polfernen Verankerungselemente (11) entgegen der Einsetzrichtung plastisch verformbar sind.

## Beschreibung

Die Erfindung betrifft eine schalenförmige, insbesondere halbkugelförmige Pfanne für eine Hüftgelenkprothese zum Einsatz im Acetabulum mit einer Vielzahl von Verankerungselementen an ihrer Außenfläche.

Zur zementfreien Verankerung einer Hüftgelenkprothese im Acetabulum muß diese mit entsprechenden Verankerungselementen versehen sein. Eine schalenförmige Pfanne der eingangs beschriebenen Art ist beispielsweise aus der EP 0 242 633 B1 bekannt. Die dort beschriebene Pfanne weist eine Außenschale auf, über die mehrere in Meridianrichtung verlaufende Schlitze verteilt sind. Im zum Äquator gelegenen Bereich der Außenschale verlaufen parallel zum Äquator mehrere Reihen von Zähnen, die durch die bereits beschriebenen Schlitze unterbrochen werden. Nach dem Einsatz im Acetabulum wird der äquatornahe, durch die genannten Schlitze unterteilte Bereich der Außenschale radial nach außen aufgespreizt, so daß die Zähne in das Gewebe eingreifen.

Die dort beschriebene Pfanne hat jedoch den Nachteil, daß sie recht kompliziert aufgebaut ist. Weiterhin muß sichergestellt werden, daß sich die Außenschale nach dem Einsetzen nicht entgegen der Aufspreizrichtung zurücksetzt und somit die Zähne ihren Halt in der Corticalis verlieren. Auch ist der Einsetzvorgang deshalb unbefriedigend, da vor dem Aufspreizen der Außenschale die Position im Acetabulum exakt festgelegt werden muß. Ein nachträgliches Korrigieren der Position der Schale im Acetabulum ist nicht möglich. Schließlich läßt sich durch die funktionsgemäß vorgesehenen Schlitze in der Außenschale keine Abdichtung zwischen der Außenschale und einer darin eingesetzten PE-Innenschale erreichen. Eine fluiddichte Abdichtung ist jedoch notwendig, da eindringende Körperflüssigkeit auch Zellen mitnimmt, die sich im Laufe der Zeit weiterbilden bzw. vermehren und wachsen. Dadurch besteht die Gefahr, daß das PE-Inlay sich von der Hüftgelenkpfanne im Laufe der Zeit löst bzw. von dieser abgehoben wird.

Aus der WO 92/18 067 ist eine Hüftgelenkpfanne bekannt, in deren Außenfläche ausschwenkbare Klingen vorgesehen sind. Diese Klingen werden nach dem Einsetzen der Pfanne im Acetabulum nach außen verschwenkt, so daß sie in die Corticalis eingreifen und entgegen der Einsetzrichtung der Pfanne wirksam sind. Die beschriebene Konstruktion ist ebenfalls aufwendig, erlaubt praktisch kein nachträgliches Korrigieren und bietet vergleichsweise wenig Angriffspunkte für ein dauerhaftes Einwachsen an der Pfanne durch Spongiose im Acetabulum.

Aufgabe der vorliegenden Erfindung ist es, eine schalenförmige Pfanne für eine Hüftgelenkprothese bereitzustellen, die wesentlich einfacher aufgebaut ist, ein leichtes Einsetzen ermöglicht, gleichzeitig einen dauerhaften Halt sicherstellt und eine einfache fluiddichte Abdichtung zwischen Außen- und Innenschale ermöglicht. Die Außenschale soll dabei sofort nach dem Einsetzen einen sicheren Halt im Acetabulum gewährleisten; dieser Halt soll jedoch durch nachfolgende Spongiosebildung und Einwachsen der schalenförmigen Pfanne im Acetabulum noch erhöht werden.

Diese Aufgabe wird durch die Merkmale des Anspruches 1 gelöst.

Die Kernidee liegt darin, daß zumindest die polfernen Verankerungselemente entgegen der Einsetzrichtung plastisch verformbar sind. Die polfernen Verankerungselemente werden beim Einsetzen widerhakenähnlich verformt und sind dann entgegen der Einsetzrichtung wirksam. Dadurch kann die Außenschale der Pfanne wesentlich einfacher aufgebaut werden. Es sind keine mechanisch beweglichen Teile mehr erforderlich. Die Gelenkpfanne kann zusammenhängend und starr ausgebildet werden, so daß die Herstellung erheblich vereinfacht ist. Die Pfanne läßt sich auch vergleichsweise einfach einsetzen, nämlich einschlagen oder eindrücken. Spezielle Verankerungsmaßnahmen sind nicht erforderlich.

Wenn die Pfanne im wesentlichen starr ausgebildet ist und die Verankerungselemente im festen Abstand voneinander angeordnet sind, können sie - anders als bei den bekannten Aufspreizsystemen - unter Belastung nicht ausweichen, sondern sich lediglich plastisch definiert verformen. Ein Austreten der Verankerungselemente senkrecht zur zugeordneten Außenfläche der Pfanne ist ausgeschlossen.

Die Verankerungselemente sind vorzugsweise als Zähne ausgebildet, die sich endseitig verjüngen und eine Spitze oder eine kurze Kante definieren. Genausogut ist es jedoch möglich, die Verankerungselemente lammellenartig oder nadelförmig auszubilden. Auch ist eine Kombination verschiedenartig gestalteter Verankerungselemente auf der Außenschale der Pfanne denkbar, beispielsweise in einer bestimmten Abfolge alternierend Zähne und Nadeln.

In einer Ausgestaltung sind die Zähne nach Art einer Pyramide mit drei oder vier Seitenflächen ausgebildet. Selbstverständlich muß die Pyramide keineswegs gleichseitig konzipiert sein. Vielmehr kann die Linie zwischen dem Mittelpunkt ihrer Basisfläche und ihrer Spitze eine von 90° verschiedenen Winkel mit der Basisflächen bzw. zugeordneten Außenfläche der Pfanne einnehmen. Auch ist es denkbar, an der polfernen Seitenfläche eine Schwächungsstelle vorzusehen, so daß eine plastische Verformung entgegen der Einsetzrichtung erleichtert wird.

Nach einer weiteren Grundidee sind die Verankerungselemente, insbesondere Zähne im äquatornahen bzw. polfernen Bereich der Pfanne vom Pol weggerichtet, wobei sie mit der Flächennormalen der jeweils zugeordneten Außenfläche der Pfanne einen Winkel α von 5° bis 60°, vorzugsweise 15° bis 45° einschließen. Dadurch wirken die Verankerungselemente schon ohne plastische Verformung als Widerhaken entgegen der Einsetzrichtung. Eine plastische Verformung der Verankerungselemente wird andererseits durch die erwähnte vorgegebene Richtung.

Im polnahen Bereich sind die Verankerungselemente vorzugsweise zum Pol hingerichtet, wobei sie mit der Flächennormalen der jeweils zugeordneten Außenfläche der Pfanne einen Winkel α von 5° bis 45°, vorzugsweise 10° bis 20° einschließen. Die zum Pol hin gerichteten Zähne bieten einen zusätzlichen Halt der Pfanne gegen seitliche Versetzungen des Pols am Boden des Acetabulums. Sie übernehmen die Aufgabe eines Zentrierungsdorns am Pol, wobei gleichzeitig dieser zentrale Dorn nicht mehr notwendig ist und die Pfanne im Bereich des Pols glatt ausgebildet werden kann.

In einer besonders bevorzugten Ausführung variiert die Ausrichtung der Verankerungselemente vom polfernen bis zum polnahen Bereich der Pfanne graduell und zwar so, daß im polfernen Bereich die Zähne vom Pol weg und im polnahen Bereich zum Pol hin gerichtet sind. In einer weiteren bevorzugten Ausgestaltung variiert die Höhe der Zähne vom äquatornahen bzw. polfernen Bereich in Richtung zum Pol hin. Dabei kann die Höhe der Zähne vom äquatornahen Bereich in Richtung zum Pol graduell abnehmen. Bevorzugt wird jedoch, daß die Höhe der Zähne vom polfernen Bereich polwärts zunächst bis auf eine maximale Zahnhöhe L zunimmt und nachfolgend in Richtung zum Pol hin wieder auf ein Minimum abnimmt.

In einer besonders bevorzugten Ausgestaltung nimmt die Zahnhöhe derart ab, daß die Außenfläche der Pfanne im Bereich des Pols glatt ausgebildet ist. Dadurch wird eine voll-flächige Anlage am Acetabulum erreicht. Die auf die Pfanne ausgeübten Kräfte werden besonders gut in den Knochen eingeleitet.

In einer weiteren bevorzugten Ausführungsform sind die Zähne in einer Schraubenlinie über die Außenfläche der Pfanne angeordnet, so daß nach dem Einsetzen der Pfanne und dem damit verbundenen plastischen Verformen der Zähne eine Niveauanpassung der Pfanne durch Ein- oder Ausschrauben der Pfanne in bzw. aus dem Acetabulum möglich ist. Durch diese Maßnahme kann die Gelenkpfanne nach dem Eindrücken bzw. Einschlagen durch Verdrehen auf das gewünschte Niveau angepaßt werden.

Zu diesem Zweck definieren die Zähne eine Schneidkante, die parallel zur Schraubenlinie ausgerichtet ist.

Schließlich weist die schalenförmige Pfanne in einer weiteren vorrteilhaften Ausgestaltung eine Innenschale aus Kunststoff, vorzugsweise aus Polyethylen (PE) auf, die fluiddicht mit der die Außenfläche definierenden Außenschale verbunden ist. Die fluiddichte Abdichtung zwischen Außen- und Innenschale läßt sich gerade deshalb in überraschend einfacher und zuverlässiger Weise erreichen, da die Außenschale zusammenhängend ohne Ausnehmungen, Schlitze oder mechanisch bewegliche Teile ausgebildet ist.

Aus diesem Grund ist es möglich, daß sich die Innenschale im wesentlichen voll-flächig an der Innenseite der Außenschale abstützt. Dadurch wird eine zuverlässige und verschleißarme Kraftübertragung zwischen Innen- und Außenschale gewährleistet. Ein "Pumpen" durch eine Relativbewegung zwischen Außen- und Innenschale wird vermieden.

Die fluiddichte Abdichtung läßt sich in überraschend einfacher Weise durch eine im äquatornahen Bereich ringförmig umlaufende Lippe und eine korrespondierende Nut erreichen, die jeweils in der Außen- oder Innenschale ausgebildet sind. Die beschriebene Lippe-Nut-Abdichtung kann gleichzeitig zur Befestigung der Innenschale an der Außenschale dienen.

Durch Einwachsen von Spongiosa zwischen die plastisch verformten Verankerungselemente, insbesondere Zähne, wird ein dauerhafter und zuverlässiger Halt der schalenförmigen Pfanne im Acetabulum erreicht. Ein Ablösen der Innenschale durch zwischen Außen- und Innenschale eindringende Körperflüssigkeit wird vermieden. Dadurch muß zumindest die Außenschale der beschriebenen schalenförmigen Pfanne für eine Hüftgelenkprothese nur noch in sehr seltenen Fällen ausgetauscht oder entfernt werden. Im Bedarfsfall läßt sich auch nur die Innenschale auswechseln. Die bereits verankerte Außenschale kann ohne weiteres eine neue Innenschale aufnehmen.

Die Erfindung wird nachstehend anhand der Beschreibung von Ausführungsbeispielen und unter Bezugnahme auf die beiliegenden Zeichnungen näher erläutert.

Hierbei zeigen
- Fig. 1: eine skizzenhafte Darstellung einer schalenförmigen Pfanne gemäß der Erfindung im Radialschnitt;
- Fig. 2a: ein polfernes Verankerungselement an der Außenfläche der Pfanne gemäß Fig. 1 vor deren Verformung in vergrößerter Ansicht;
- Fig. 2b: ein polfernes Verankerungselement an der Außenfläche gemäß Fig. 1 nach deren Verformung in vergrößerter Ansicht;
- Fig. 3a: eine andere Ausgestaltung eines polfernen Verankerungselements vor der Verformung entsprechend der Ansicht nach Fig. 2a;
- Fig. 3b: das Verankerungselement nach Fig. 3a nach der Verformung;
- Fig. 4a: eine weitere Ausgestaltung eines polfernen Verankerungselementes vor der Verformung entsprechend der Ansicht nach Fig. 2a;
- Fig. 4b: das Verankerungselement nach Fig. 4a nach der Verformung; und
- Fig. 5: einen Teil der schalenförmigen Pfanne gemäß Fig. 1 im Radialschnitt und vergrößertem Maßstab.

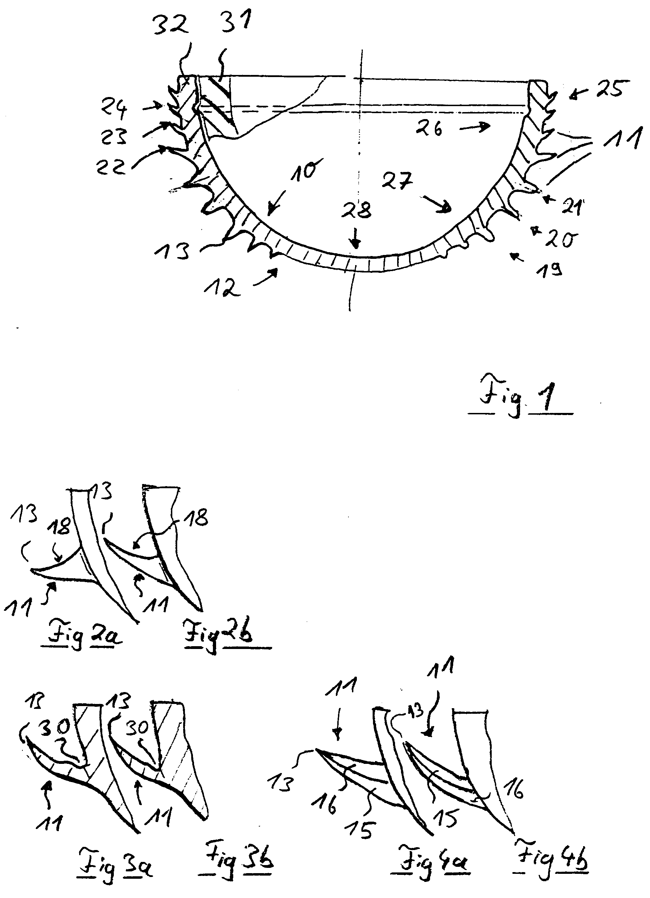

In Fig. 1 ist eine nahezu schalenförmige Pfanne 10 für eine Hüftgelenkprothese zum Einsatz im Acetabulum gezeigt, die eine Außenschale 32 aus körperverträglichem Metall, vorzugsweise aus Reintitan oder Titanlegierung und eine Innenschale 31 aus Kunststoff aufweist. Die Innenschale 31 wird üblicherweise aus Polyethylen (PE) hergestellt, das hinreichend körperverträglich ist und die an die Innenschale einer künstlichen Gelenkpfanne gestellten mechanischen Anforderungen erfüllt. Die aus Reintitan oder Titanlegierung hergestellte Außenschale weist an ihrer Außenfläche 12 eine Vielzahl von Zähnen 11 auf. Die Zähne 11 sind in mehreren auf der Außenfläche 12 der Pfanne 10 äquatorparallel umlaufenden Reihen 19, 20, 21, 22, 23, 24, 25 angeordnet.

Die Zähne 11 bestehen ebenfalls aus demselben Werkstoff wie die Außenschale 32 bzw. sind integral mit der Außenschale 32 hergestellt.

Zumindest die polfernen bzw. äquatornahen Zähne sind derart ausgebildet, daß sie beim Einsetzen entgegen der Einsetzrichtung plastisch verformbar sind.

Damit die gewünschte plastische Verformung beim Einsetzen erreicht wird, ist die Außenschale zusammenhängend und relativ starr ausgebildet. Die Vielzahl von Zähnen 11 sind auf der Außenfläche 12 der Außenschale 32 im festen Abstand voneinander angeordnet. Die Zähne unterschiedlicher Reihen 19, ..., 25 können jeweils direkt übereinander angeordnet sein, wie die Schnittansicht von Fig. 1 erkennen läßt. Die Zähne unterschiedlicher Reihen 19, ..., 25 können alternativ auch von einer Reihe zur nächsten versetzt zueinander angeordnet sein. In einer weiter alternativen Ausgestaltung können sich die Zähne auch vollkommen beliebig bzw. willkürlich auf der Außenfläche der Pfanne befinden.

Die Zähne selbst können unterschiedliche Formen aufweisen. Auch kann es zweckmäßig sein, die Form der Zähne vom polfernen Bereich in Richtung zum Pol hin zu variieren. Wie bereits erwähnt, werden zumindest die polfernen Zähne z. B. die Zähne der beiden dem Äquator nächstgelegenen Zahnreihen derart ausgebildet, daß sie entgegen der Einsetzrichtung plastisch verformbar sind. Dies läßt sich durch ein entsprechendes Verhältnis von Zahnhöhe zu Zahnstärke einstellen. Ein hoher Zahn ist beim Eindrücken oder Einschlagen der Pfanne 10 wesentlich höheren Biegekräften ausgesetzt als ein vergleichsweise niedriger Zahn. Wenn der entsprechende Zahn nicht zu stark ausgebildet ist, wird er den auftretenden Biegekräften beim Einschlagen oder Eindrücken der Außenschale ausweichen und sich plastisch entgegen der Einsetzrichtung verformen.

In den Fig. 2a, 2b, 3a, 3b, 4a, 4b sind drei unterschiedliche Zahnformen für die polfernen Verankerungszähne jeweils in schematischer Ansicht vor und nach dem Einsetzen der Pfanne bzw. Außenschale 32 dargestellt.

Die plastische Deformaierbarkeit soll vor allem bei den polfernen Zähnen 11 gegeben sein. Die Deformierbarkeit der Zähne 11 kann vom polnahen Bereich 27 der Außenschale 32 zum polfernen Bereich 26 graduell zunehmen. Alternativ ist es möglich, daß nur die erste 25 oder die ersten beiden polfernen Zahnreihen 25, 24 deformierbare Zähne aufweisen. Die näher zum Pol 28 gelegenen Zähne 11 sind in diesem Fall relativ starr ausgebildet.

In Fig. 2a ist ein Zahn gezeigt, der bereits vor dem Einschlagen an der Spitze 13 etwas vom Pol 28 weg gerichtet ist. Außerdem definiert der Zahn 11 an seiner polabgewandten Seite eine Bogenlinie 18, die zum Äquator hin gekrümmt ausgebildet ist. Nach dem Einschlagen (Fig. 2b) ist der Zahn 11 entgegen der Einsetzrichtung plastisch verformt, so daß er eine Art Widerhaken definiert.

In Fig. 3a ist ein Zahn 11 mit einer Schwächungsstelle 30 gezeigt, die auf der polabgewandten Seite des Zahns angeordnet ist. Diese Schwächungsstelle 30 begünstigt ein Verformen bzw. Einknicken des Zahns 11 beim Einsetzen, so daß die plastische Verformung in die gewünschte Richtung erleichtert wird.

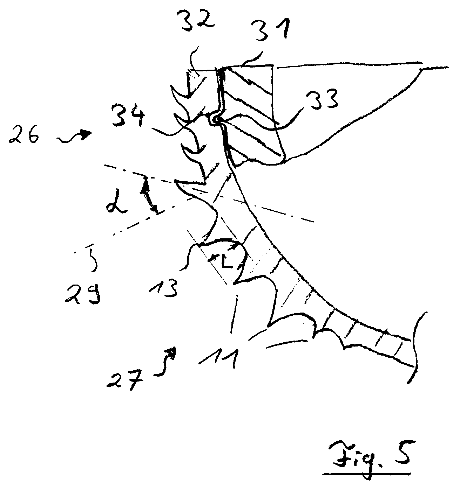

Bei dem in den Fig. 4a und Fig. 4b skizzierten Ausführungsbeispiel ist der Zahn 11 pyramidenähnlich ausgebildet. Er weist Seitenflächen 15, 16 auf sowie zwei weitere in den Fig. 4a und 4b nicht erkennbare Seitenflächen. Wählt man die Flächen der polzugewandten 15 und der polabgewandten Fläche 16 relativ zu den dazwischenliegenden Seitenflächen sehr groß, so nimmt der Zahn 11 eine klingenähnliche Form an. Durch die pyramidenähnliche Ausbildung wird eine plastische Verformung in einer zur Einsetzrichtung schrägen Richtung weitgehend unterdrückt. Die flächige Ausbildung des Zahns an seiner polabgewandten Seite erhöht die Haltekraft unmittelbar nach dem Einsetzen der Außenschale und begünstigt die nachfolgende Verankerung durch einwachsende Spongiosa.

Wie Fig. 5 erkennen läßt sind jedoch nur die polfernen Zähne vom Pol weggerichtet. Dabei schließen sie mit der Flächennormalen 29 der jeweils zugeordneten Außenfläche der Pfanne 10 einen Winkel α ein, der in Richtung vom Äquator zum Pol hin abnimmt. Bei der gezeigten Ausführungsform beträgt der Winkel α in der vom äquatornahen Rand der Außenschale 32 vierten Zahnreihe 22 circa 40°. Die polnäheren Zahnreihen 21, 20, 19 sind entweder senkrecht zu ihren jeweils zugeordneten Außenflächen der Pfanne 10 ausgerichtet oder sogar zum Pol hin gerichtet. Allerdings liegen hier die in Betracht kommenden Winkel lediglich zwischen 0° und 45°, wobei ein Bereich zwischen 10° und 20° bevorzugt wird.

Wie Fig. 5 ebenfalls erkennen läßt, variiert auch die Höhe der Zähne 11 vom polfernen Bereich 26 zum Pol 28 hin. Im polfernen Bereich 26 der Außenschale 32 nimmt die Zahnlänge von einem bestimmten Ausgangswert an zunächst bis auf eine maximale Zahnhöhe L zu und fällt dann in Richtung zum Pol auf 0 ab. Der Abfall der Zahnhöhe auf 0 geschieht im polnahen Bereich 27 in ausreichendem Abstand vom Pol, so daß der Bereich um den Pol 28 zahnfrei ist. In diesem zahnfreien Bereich ist die Außenschale 32 der Pfanne 10 glatt ausgebildet. Dies ermöglicht eine flächige Anlage der Außenschale im Acetabulum und damit eine wirksame Krafteinleitung in den Knochen. Die Höhe der Zähne im polfernen Bereich hängt stark von der jeweils gewählten Zahnform ab. Typischerweise liegt sie in einem Bereich von 0,7 mm bis 173 mm, wobei Höhen von 0,9 mm bis 1,1 mm bevorzugt werden. Die maximale Zahnhöhe L beträgt zwischen 3,0 mm und 4,0 mm, vorzugsweise zwischen 2,7 mm und 3,3 mm.

In einer weiteren bevorzugten Ausgestaltung (nicht dargestellt) folgen die Reihen 19, ..., 25 der Zähne 11 einer Schraubenlinie über die Außenfläche 12 der Außenschale 32. Somit ist es möglich, durch leichtes Verdrehen der Außenschale 32 nach dem Einsetzen eine Niveauanpassung derselben bzw. der gesamten Pfanne durch Ein- oder Ausschrauben der Außenschale 32 in bzw. aus dem Acetabulum zu erreichen. In dieser Ausgestaltung ist es zweckmäßig, daß die Zähne 11 eine kurze Schneidkante definieren, die parallel zur Schraubenlinie ausgerichtet ist. Eine solche Schneidkante läßt sich beispielsweise durch leichte Modifikation der in Fig. 4a und 4b skizzierten Zahnform erzielen.

Dadurch, daß die Außenschale 32 der beschriebenen Pfanne 10 zusammenhängend und ohne Schlitze oder mechanisch bewegliche Verankerungselemente ausgebildet ist, läßt sich auf relativ einfache Weise eine fluiddichte Abdichtung zwischen Außenschale 32 und Innenschale 31 erreichen. Hierzu ist an der Innenseite der Außenschale 32 eine umlaufende Nut 34 vorgesehen, in die eine ringförmige Lippe 33 an der Außenseite der Innenschale 31 eingreift. Die beschriebene Abdichtung zwischen Außenschale 32 und Innenschale 31 kann gleichzeitig als Befestigung der Innenschale 31 an der Außenschale 32 dienen. Durch die zusammenhängende Ausbildung der Außenschale 32 und die vorstehend beschriebene Abdichtung läßt sich eine im wesentlichen voll-flächige Anlage der Innenschale 31 an der Außenschale 32 erreichen. Dadurch ist eine äußerst wirksame Kraftübertragung zwischen Innenschale 31 und Außenschale 32 gegeben. Die starre Ausbildung der Außenschale 32 gewährleistet darüber hinaus eine exakte Paßform der Innenschale 31.

## Patentansprüche

1. Schalenförmige, insbesondere halbkugelförmige Pfanne für eine Hüftgelenkprothese zum Einsatz im Acetabulum mit einer Vielzahl von Verankerungselementen (11) an ihrer Außenfläche (12),
**dadurch gekennzeichnet,** daß
zumindest die polfernen Verankerungselemente (11) entgegen der Einsetzrichtung plastisch verformbar sind.

2. Schalenförmige Pfanne nach Anspruch 1,
**dadurch gekennzeichnet,** daß
sie starr ausgebildet ist, und daß die Vielzahl von Verankerungselementen (11) in gleichbleibenden Abstand voneinander angeordnet sind.

3. Schalenförmige Pfanne nach einem der Ansprüche 1 bis 2,
**dadurch gekennzeichnet,** daß
die Außenfläche (12) der schalenförmigen Pfanne (10) zusammenhängend und aus körperverträglichem Material, wie Metall, insbesondere Reintitan oder Titanlegierung, hergestellt ist.

4. Schalenförmige Pfanne nach einem der Ansprüche 1 bis 3,
**dadurch gekennzeichnet,** daß
die Verankerungselemente (11) ebenfalls aus Metall, insbesondere aus Reintitan oder Titanlegierung bestehen und integral mit der Außenfläche (12) der Pfanne (10) ausgebildet sind.

5. Schalenförmige Pfanne nach einem der Ansprüche 1 bis 4,
**dadurch gekennzeichnet,** daß
die Verankerungselemente als Zähne (11) ausgebildet sind, die sich endseitig verjüngen und eine Spitze (13) oder kurze Kante definieren.

6. Schalenförmige Pfanne nach Anspruch 5,
**dadurch gekennzeichnet,** daß
die Zähne (11) nach Art einer Pyramide mit drei oder vier Seitenflächen (15, 16, 17, 18) ausgebildet sind.

7. Schalenförmige Pfanne nach Anspruch 5 oder 6,
**dadurch gekennzeichnet,** daß
die Zähne (11) in mehreren, an der Außenfläche (12) der Pfanne (10) etwa äquatorparallel umlaufenden Reihen (19,..., 25) angeordnet sind.

8. Schalenförmige Pfanne nach Anspruch 5 oder 6,
**dadurch gekennzeichnet,** daß
die Zähne (11) beliebig auf der Außenfläche (12) der Pfanne (10) verteilt angeordnet bzw. ausgebildet sind.

9. Schalenförmige Pfanne insbesondere nach einem der Ansprüche 1 bis 8,
**dadurch gekennzeichnet,** daß
die Verankerungselemente, insbesondere Zähne (11) im äquatornahen bzw. polfernen Bereich (26) der Pfanne (10) vom Pol (28) weg gerichtet sind, wobei sie mit der Flächennormalen (29) der jeweils zugeordneten Außenfläche der Pfanne (10) einen Winkel α von 5° bis 60°, vorzugsweise 15° bis 45° einschließen.

10. Schalenförmige Pfanne insbesondere nach einem der Ansprüche 1 bis 9,
**dadurch gekennzeichnet,** daß
die Verankerungselemente, insbesondere Zähne (11) im polnahen Bereich (27) der Pfanne (10) zum Pol (28) hin gerichtet sind, wobei sie mit der Flächennormalen (29) der jeweils zugeordneten Außenfläche der Pfanne (10) einen Winkel α von 0° bis 45°, vorzugsweise 10° bis 20° einschließen.

11. Schalenförmige Pfanne insbesondere nach einem der Ansprüche 1 bis 10,
**dadurch gekennzeichnet,** daß
die Ausrichtung der Verankerungselemente, insbesondere Zähne (11) vom polfernen (26) bis zum polnahen Bereich (27) der Pfanne (10) variiert und zwar so, daß im polfernen Bereich (26) die Zähne (11) vom Pol (28) weg gerichtet und im polnahen Bereich (27) zum Pol (28) hin gerichtet sind.

12. Schalenförmige Pfanne nach einem der Ansprüche 5 bis 11,
**dadurch gekennzeichnet,** daß
die Höhe der Zähne (11) vom äquatornahen bwz polfernen Bereich (26) in Richtung zum Pol (28) hin variiert.

13. Schalenförmige Pfanne nach einem der Ansprüche 5 bis 12,
**dadurch gekennzeichnet,** daß
die Höhe der Zähne (11) vom polfernen Bereich (26) polwärts zunächst bis auf eine maximale Zahnhöhe L zunimmt und nachfolgend in Richtung zum Pol (28) hin wieder auf ein Minimum abnimmt.

14. Schalenförmige Pfanne nach einem der Ansprüche 5 bis 13,
**dadurch gekennzeichnet,** daß
die Außenfläche (12) der Pfanne (10) im Bereich des Pols (28) glatt ausgebildet ist.

15. Schalenförmige Pfanne nach einem der Ansprüche 5 bis 14,
**dadurch gekennzeichnet,** daß
die Höhe der Zähne (11) im polfernen Bereich (26) 0,7mm bis 1,3mm, vorzugsweise 0,9mm bis 1,1mm beträgt.

16. Schalenförmige Pfanne nach einem der Ansprüche 5 bis 15,
**dadurch gekennzeichnet,** daß
die maximale Zahnhöhe L 2mm bis 4mm, vorzugsweise 2,7mm bis 3,3mm beträgt.

17. Schalenförmige Pfanne nach einem der Ansprüche 7 bis 16,
**dadurch gekennzeichnet,** daß
die Reihen (19, ..., 25) der Zähne (11) einer Schraubenlinie S über die Außenfläche (12) der Pfanne (10) folgen, so daß nach dem Einsetzen der Pfanne (10) und dem damit verbundenen plastischen Verformen der Zähne (11) eine Niveauanpassung der Pfanne (10) durch Ein- oder Ausschrauben derselben in bzw. aus dem Acetabulum hinein bzw. heraus möglich ist.

18. Schalenförmige Pfanne nach Anspruch 17,
**dadurch gekennzeichnet,** daß
die Zähne (11) endseitig eine Schneidkante (14) definieren, die der durch die Reihen (19, ..., 25) festgelegten Schraubenlinie S folgt.

19. Schalenförmige Pfanne nach einem der vorhergehenden Ansprüche,
**dadurch gekennzeichnet,** daß
sie weiterhin eine Innenschale (31) aus Kunststoff, insbesondere Polyethylen (PE) aufweist, die fluiddicht mit einer die Außenfläche (12) definierenden Außenschale (32) der Pfanne (10) verbunden ist.

20. Schalenförmige Pfanne nach Anspruch 19,
**dadurch gekennzeichnet,** daß
sich die Innenschale (31) im wesentlichen voll-flächig an der Innenseite der Außenschale (32) abstützt.

21. Schalenförmige Pfanne nach Anspruch 19 oder 20,
**dadurch gekennzeichnet,** daß
die fluiddichte Abdichtung zwischen Innenschale (31) und Außenschale (32) durch eine äquatornah angeordnete ringförmige Lippe (33) an der Außenseite der Innenschale (31), die in eine korrespondierende Nut (34) in der Innenseite der Außenschale (32) eingreift, gebildet ist.

22. Schalenförmige Pfanne nach Anspruch 19 oder 20,
**dadurch gekennzeichnet,** daß
die fluiddichte Abdichtung zwischen Innenschale (31) und Außenschale (32) durch eine äquatornah angeordnete ringförmige Lippe an der Innenseite der Außenschale (32), die in eine korrespondierende Nut in der Außenseite der Innenschale (31) eingreift, gebildet wird.
